Europäisches Patentamt

European Patent Office

Office européen des brevets

(11) Publication number: **0 492 552 A1**

# EUROPEAN PATENT APPLICATION

(21) Application number: **91122026.7**

(22) Date of filing: **20.12.91**

(51) Int. Cl.⁵: **C12P 21/08**, C12N 15/06, C12N 5/20, G01N 33/577, G01N 33/74

(30) Priority: **21.12.90 JP 418284/90**
**27.06.91 JP 250138/91**

(43) Date of publication of application:
**01.07.92 Bulletin 92/27**

(84) Designated Contracting States:
**AT BE CH DE DK FR GB IT LI NL SE**

(71) Applicant: **DAIICHI PHARMACEUTICAL CO., LTD.**
**14-10, Nihonbashi 3-chome**
**Chuo-ku, Tokyo 103(JP)**

(72) Inventor: **Sakano, Katsuichi, c/o Daiichi Pharm. Co., Ltd.**
**Tokyo R & D Center, 16-13, Kitakasai 1-chome**
**Edogawa-ku, Tokyo(JP)**
Inventor: **Marumoto, Yasumasa, c/o Daiichi Pharm. Co., Ltd.**
**Tokyo R & D Center, 16-13, Kitakasai 1-chome**
**Edogawa-ku, Tokyo(JP)**
Inventor: **Enjoh, Tomoko, c/o Daiichi Pharm. Co., Ltd.**
**Tokyo R & D Center, 16-13, Kitakasai 1-chome**
**Edogawa-ku, Tokyo(JP)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

(54) **Anti-IGF-II monoclonal antibody.**

㊵ Anti-human insulin-like growth factor II monoclonal antibodies having extremely high antigenic specificity and differing in recognition site, and a process for preparing the same. The monoclonal antibodies which recognize human insulin-like growth factor II, but do not recognize human insulin-like growth factor I are obtained by using, as an antigen, a polypeptide derived from human insulin-like growth factor II by replacing the 43rd amino acid with leucine. The monoclonal antibodies are obtained from monoclonal antibody-producing hybridoma cells.

## FIELD OF THE INVENTION

This invention relates to monoclonal antibodies specific for human insulin-like growth factor II and a process for preparing the same.

## BACKGROUND OF THE INVENTION

Human insulin-like growth factor II (hereinafter abbreviated as IGF-II) is a polypeptide composed of 67 amino acids and exhibits insulin-like activities. It is known to widely occur in the human body, such as in blood and bone tissues. IGF-II is highly homologous to human insulin-like growth factor I (hereinafter abbreviated as IGF-I) in amino acid sequence, and many of the anti-IGF-I antibodies and the anti-IGF-II antibodies reported have cross-reactivity (J. Clin. Invest., Vol. 60, pp. 646-657 (1977); J. Clin. Endocrinol. Metab., Vol. 50, pp. 405-406 (1980); and J. Clin. Invest., Vol. 68, pp. 1321-1330 (1981)).

Hence, achievement of the precise determination or analysis of IGF-II or isolation of IGF-II from a living body requires a monoclonal anti-IGF-II antibody having higher specificity.

## SUMMARY OF THE INVENTION

An object of the present invention is to provide various anti-IGF-II monoclonal antibodies having extremely high antigenic specificity and differing in recognition sites and another object of this invention is to provide a process for preparing the same.

An appropriate combination of these antibodies is expected to be useful and preferable for enzyme immunoassay techniques, etc. and thereby to make precise determination of IGF-II feasible. Further, such useful antibodies are also applicable to various analyses of IGF-II, such as Western blotting analysis, or purification of IGF-II.

Thus, the present invention provides monoclonal antibodies which specifically bind to IGF-II but do not bind to IGF-I.

Another object of the present invention is to provide a monoclonal antibody that specifically recognizes the amino acid sequence: Phe-Ser-Arg-Pro-Ala-Ser-Arg-Val-Ser-Arg-Arg-Ser-Arg-Gly (SEQ ID NO:1), and monoclonal antibodies that also specifically recognize IGF-II but not IGF-I and have different epitope specificity.

Still another object of the present invention is to provide a process for preparing a monoclonal antibody belonging to immunoglobulin class IgG that specifically binds to IGF-II but does not bind to IGF-I, comprising immunizing a mammal using, as an antigen, a polypeptide having an amino acid sequence (SEQ ID NO:2) Ala-Tyr-Arg-Pro-Ser-Glu-Thr-Leu-Cys-Gly-Gly-Glu-Leu-Val-Asp-Thr-Leu-Gln-Phe-Val-Cys-Gly-Asp-Arg-Gly-Phe-Tyr-Phe-Ser-Arg-Pro-Ala-Ser-Arg-Val-Ser-Arg-Arg-Ser-Arg-Gly-Ile-Leu-Glu-Glu-Cys-Cys-Phe-Arg-Ser-Cys-Asp-Leu-Ala-Leu-Leu-Glu-Thr-Tyr-Cys-Ala-Thr-Pro-Ala-Lys-Ser-Glu (hereinafter referred to as polypeptide-2); preparing a hybridoma from antibody-producing cells of the immunized mammal and myeloma cells; selecting a desired hybridoma; culturing the hybridoma; and collecting the antibody.

The present invention further provides the monoclonal antibody obtainable by the process of the present invention.

The amino acid sequence of polypeptide-2 (SEQ ID NO:2) can be used in the present invention is the same as that of IGF-II except that the 43rd amino acid is replaced with leucine. By the use of this polypeptide as an antigen, several types of monoclonal antibodies can be obtained which specifically bind to IGF-II and do not bind to IGF-I.

## BRIEF DESCRIPTION OF THE DRAWINGS

Figures 1 and 2 each illustrate a construction diagram for obtaining recombinant expression virus.

Figure 3 shows reaction specificity of each antibody according to the present invention.

Figure 4 shows a HPLC elution pattern of peptide fragments derived from IGF-II by pepsin digestion.

Figure 5 shows a standard curve of competition ELISA using antibody according to the present invention.

Figure 6 shows a result of Western blotting analysis using antibody according to the present invention.

## DETAILED DESCRIPTION OF THE INVENTION

The monoclonal antibodies obtainable by the present invention belong to the IgG class and specifically bind IGF-II but not recognize IGF-I. For example, the antibody designated by the number 2H11 recognizes an amino acid sequence: Phe-Ser-Arg-Pro-Ala-Ser-Arg-Val-Ser-Arg-Arg-Ser-Arg-Gly. There are also obtained monoclonal antibodies which belong to the IgG class and recognize IGF-II but do not bind IGF-I and which are different from 2H11 in the binding characteristics, for example, those designated by the numbers 2B11, ID5 and ID9. A combination of two of these monoclonal antibodies makes it possible to measure the concentration of IGF-II by double antibodies sandwich technique.

Monoclonal antibody 2H11, 2B11, ID5 or ID9 can be prepared by using monoclonal antibody-producing hybridoma 2H11, hybridoma 2B11, hybridoma ID5, or hybridoma ID9.

The individual operations involved in the process of the present invention, that is, immunization of animals, recovery of antibody-producing cells, cell fusion for obtaining a hybridoma population, recovery of the desired hybridoma, and culturing of the hybridoma to obtain a monoclonal antibody, can be carried out by generally employed techniques. For details, reference can be made, e.g., in Nature, Vol. 256, pp. 495-497 (1975) and Proc. Natl. Acad. Sci. U.S.A., Vol. 78, pp. 5122-5126 (1981).

Specific embodiments of the process for preparing monoclonal antibodies according to the present invention will be explained below.

A. Preparation of Antigen:

Polypeptide-2 which can be used as an antigen in the present invention is prepared with herein described modifications of the process for preparing IGF-II reported, e.g., in Marumoto, et al., J. gen. Virol., Vol. 68, pp. 2599-2606 (1987) using site-directed mutagenesis techniques (see, e.g., Sambrook, J. et al., Molecular Cloning, 2nd Ed., Vol. 2, 15.51-15.80 (1989) and Kunkel, T.A., et al., Methods in Enzymology, Vol. 154, pp. 367-382 (1987), the entire contents of which are herein incorporated by reference.).

B. Immunization of Mouse with Polypeptide-2:

Female BALB/c mice can be used. Other strains of mice and rats are also usable, as known in the art. It should be noted that the immunization plan and the sensitizing concentration of polypeptide-2 are designed so as to produce a sufficient amount of sensitized-lymphocytes. For example, 0.1 mg of a polypeptide-2/bovine serum albumin (BSA) conjugate is subcutaneously administered to a mouse 4 times at 2 to 3 week intervals, and 0.1 mg of the conjugate is intraperitoneally administered for final immunization. Several days after the final immunization, spleen cells are taken out of the immunized mammal for cell fusion.

C. Cell Fusion:

The spleen of the thus immunized mammal can be sterilely excised, and a spleen cell suspension then prepared. The spleen cells are fused with appropriate mouse myeloma cells of an established line in the presence of an appropriate cell fusion accelerator. A preferred ratio of spleen cells to myeloma cells ranges from about 20:1 to 2:1 (cell number), and about 1 ml of a fusion medium is suitably used per about $10^8$ spleen cells.

The terminology "fusion medium" as used herein means solutions, etc., containing a cell fusion accelerator for cell maintenance. Myeloma cells that can be used for cell fusion for obtaining hybridoma cells include P3 X63 Ag8 cells, P3 x63 Ag8 U1 cells, P3 X63 Ag8 653 cells and NS I cells of mouse origin and 210 RCY Ag1.2.3 cells of rat origin. In Examples hereinafter described, P3 X63 Ag8 U1 cells (see Current Topics in Microbiology, Vol. 81, pp. 1-7 (1978), the entire contents of which are herein incorporated by reference.) were used.

Usable cell fusion accelerators include, e.g., polyethylene glycol, dimethyl sulfoxide, and L-arginine. While polyethylene glycol having an average molecular weight of about 1000 to 6000 is usually employed, other fusion accelerators can also be employed, and two or more fusion accelerators can be used in combination. The media for dissolving the fusion accelerator include distilled water, phosphate-buffered physiological saline, and various media for cell culturing. In the following Examples, distilled water having dissolved therein polyethylene glycol, having an average molecular weight of about 1500, was used as a fusion medium.

Cell fusion may also be carried out by an electrical cell fusion technique using a cell fusion apparatus (see FEBS Letters, Vol. 147, pp. 64-67 (1982)) or cell fusion technique using HVJ (Sendai virus) (see Nature, Vol. 256, pp. 495-497 (1975), the entire contents of which are herein incorporated by reference.).

D. Selection of Hybridoma:

The mixture obtained by cell fusion containing non-fused spleen cells, non-fused mouse myeloma cells, and fused cells (hybridoma) can be diluted with a selection medium which does not maintain the non-fused mouse myeloma cells, such as a HAT medium, and then incubated for a period sufficient to kill the non-fused cells (e.g., about 1 week). In the selection medium, the non-fused mouse myeloma cells die. Being non-tumor cells, the non-fused spleen cells also die in the medium after a certain period (about 1 week). However, the fused hybridoma cells survive in the above-described selection medium.

E. Assay of Antibody Titer:

The antibody titer during mouse immunization, and detection and confirmation of the antibody produced by the thus selected hybridoma cells, can then be assayed by an enzyme linked immunosorbent assay (ELISA) for antipolypeptide-2 antibodies.

F. Selection of Antibody-Producing Hybridoma Cells:

Selection of antibody-producing hybridoma cells can be achieved by cloning of those cells capable of producing the desired antibody by an appropriate method, such as limiting dilution method.

G. Production of Monoclonal Antibodies:

A desired monoclonal antibody can be produced by two methods: one comprising culturing each type of hybridoma cells in an appropriate medium for a proper period of time and recovering the antibody produced by the hybridoma cells from the supernatant liquid of the culture; and the other comprising intraperitoneally administering the hybridoma cells to a mouse and, after a proper period of time, recovering the antibody produced by the hybridoma cells from the blood or ascites of the host mouse. Any type of hybridoma cells in the present invention is suitable for either of these two antibody production methods.

H. Purification of Monoclonal Antibody:

The monoclonal antibody-containing culture liquid or ascites obtained in G. above is purified by known techniques. For example, the ascites is subjected to affinity chromatography using a protein A column and then concentrated by an ultrafiltration.

There are thus obtained monoclonal antibodies which belong to immunoglobulin IgG and recognize IGF-II but do not recognize IGF-I, such as monoclonal antibodies 2H11, 2B11, ID5, and ID9.

The present invention is now illustrated in greater detail by way of Examples, but it should be understood that the present invention is not deemed to be limited thereto. All the percents are given by weight unless otherwise indicated.

EXAMPLE 1: Preparation of Antigen

(1) Preparation of DNA Oligomer for Mutation Introduction:

A DNA oligomer for mutation introduction to replace the DNA sequence that encodes for amino acid 43 of IGF-II with a sequence encoding a leucine, having a base sequence (SEQ ID NO:3) TTCTTCGAGGATACCTC (heptadecamer) (hereinafter referred to as Mut-IGF-II-02), was synthesized by means of a DNA synthesizing machine and purified by high performance liquid chromatography (HPLC). The 5'-terminal of the purified Mut-IGF-II-02 (10 pmol) was phosphorylated by T4 polynucleotide kinase and used for synthesis of a complementary chain.

(2) Preparation of Single-Stranded DNA Containing Deoxyuridine:

Plasmid pIGF002 (see J. gen. Virol., Vol. 68, pp. 2599-2606 (1987); the Escherichia coli strain having this plasmid, E. coli K12 MC1061 IGF-002, was deposited with Fermentation Research Institute, Agency of Industrial Science & Technology (address; 1-3, Higashi 1 chome, Yatabe-machi, Tsukuba-gun, Ibaraki-ken 305, Japan) on November 6, 1985, MITI under receipt number FERM BP-933) was partially digested with restriction enzymes, EcoRI and SalI, to separate an IGF-II gene DNA fragment having about 230 base pairs

4

(bp), and the resulting DNA fragment was inserted into pUC19 cleaved with EcoRI and SalI. The resulting plasmid pUCIGF-II was cleaved with EcoRI and HindIII and treated with mung bean nuclease to separate an IGF-II gene DNA fragment of about 230 bp. The fragment was then inserted into plasmid pUC119 cleaved with SmaI to obtain plasmid p119-IGF-II (14) with its region coding the IGF-II N-terminal on the HindIII side and plasmid p119-IGF-II (24) in which the IGF-II gene is inversely inserted. p119-IGF-II (14) was transfected into Escherichia coli MV1184. The strain was cultured in 2xYT medium (containing ampicillin, tetracycline, and streptomycin), and the culture was infected with helper phage M13K07 (m.o.i. 2-10). After allowing the culture to stand at 37°C for 30 minutes, kanamycin was added thereto, and culturing was continued overnight. The culture was centrifuged to obtain the supernatant liquid (phage liquid). The phage liquid was added to an Escherichia coli BW 313 culture (LB) for infection (m.o.i. 2-10). After allowing the culture to stand at 37°C for 30 minutes, kanamycin was added to the culture, and the culturing was further continued at 37°C overnight. The culture was centrifuged, and to the supernatant liquid was added 25 ml of a 2.5 M NaCl/20% polyethylene glycol 6000 solution. After allowing the system to stand at room temperature for 10 minutes, the system was subjected to centrifugation to recover a precipitate. The precipitate was dissolved in 5 ml of 1 mM EDTA/10 mM Tris•HCl buffer (pH=8) (hereinafter referred to as TE buffer) and treated successively with phenol; a mixture of phenol, chloroform and isoamyl alcohol (25:24:1 by volume); and a mixture of chloroform and isoamyl alcohol (24:1 by volume) in this order. The aqueous layer was recovered, and 500 μl of 3 M sodium acetate (pH=8.0) and 5 ml of isopropyl alcohol were added thereto, followed by stirring. The mixture was centrifuged, and the resulting precipitate was washed with 70% ethanol and dried under reduced pressure. The residue was dissolved in TE buffer to prepare a solution of single-stranded DNA containing deoxyuridine (hereinafter referred to as p119-IGF-II•dUssDNA).

(3) Preparation of Mutation-Introduced Plasmid p119-M02-IGF-II:

In an annealing buffer, 0.02 pmol of p119-IGF-II•dUssDNA and 1 pmol of the 5'-phosphorylated DNA oligomer for mutagenesis (Mut-IGF-II-02) were mixed, and the mixture was allowed to stand at 65°C for 15 minutes and then at 37°C for 15 minutes. To the system were added 255 μl of an elongation buffer (Mutan™-K kit produced by Takara Shuzo Co., Ltd.), E. coli DNA ligase (60U), and T4 DNA polymerase (1U), followed by allowing the mixture to stand at 25°C for 2 hours. Then, 3 μl of 0.2 M EDTA (pH=8.0) was added thereto, the system was allowed to stand at 65°C for 5 minutes, and the reaction was stopped.

Thirty microliters of E. coli BMH71-18 mutS competent cells and 3 μl of the above-prepared DNA solution were mixed and allowed to stand at 0°C for 30 minutes, then at 42°C for 45 seconds, and finally at 0°C for 2 minutes. To the mixture was added 1 ml of 2xYT medium (containing ampicillin and kanamycin), and the mixture was shaken at 37°C overnight, followed by centrifugation. The supernatant liquid was recovered, and a 20 μl aliquot thereof was mixed with 80 μl of a culture of E. coli MV 1184. An adequate amount of the culture was spread on an LB-agar medium (containing ampicillin) and cultured at 37°C. An adequate number of single colonies were taken out, plasmid DNA was prepared in a conventional manner, and the base sequence at the site where mutation had been introduced was confirmed. There was thus obtained plasmid p119-M02-IGF-II having inserted therein a mutation-introduced IGF-II gene wherein the sequence encoding amino acid 43 of IGF-II is replaced with a sequence encoding a leucine.

(4) Preparation of Plasmid pBM-M02-IGF-II for Preparation of Recombinant Virus:

pFIGF-II 120 (see J. gen. Virol., Vol. 68, pp. 2599-2606 (1987)) was digested with EcoRI and then treated with DNA polymerase (Klenow fragment) in the presence of dNTPs to make the terminals blunt-ended, and a DNA fragment of about 10 kbp was separated and extracted by agarose gel electrophoresis. DNA was recovered by a DEAE filter, washed and dried in a conventional manner, dissolved in 200 μl of 1M Tris•HCl, and treated with bacterial alkaline phosphatase (a product of Takara Shuzo Co., Ltd., hereinafter abbreviated as BAP) to obtain a vector. The resulting vector was dissolved in a suitable amount of TE buffer to prepare a ligation sample.

The mutation-introduced plasmid p119-M02-IGF-II was digested with NcoI and EcoRI and then treated with DNA polymerase (Klenow fragment) in the presence of dNTPs to make the terminals blunt-ended. A DNA fragment of about 220 bp was separated and extracted by agarose gel electrophoresis, and the fragment was recovered by a DEAE filter, washed, dried, and dissolved in a suitable amount of TE buffer to prepare a ligation sample.

The above-prepared vector and DNA fragment were ligated by using T4 DNA ligase. E. coli MC1061 was transformed by using the resulting reaction liquid. A part of the culture was spread on an LB-agar medium containing ampicillin and cultured at 37°C overnight. An adequate number of the resulting single colonies was taken out, and the base sequence of the plasmid was analyzed and confirmed in a conventional manner to obtain E. coli having plasmid pBM-M02-IGF-II.

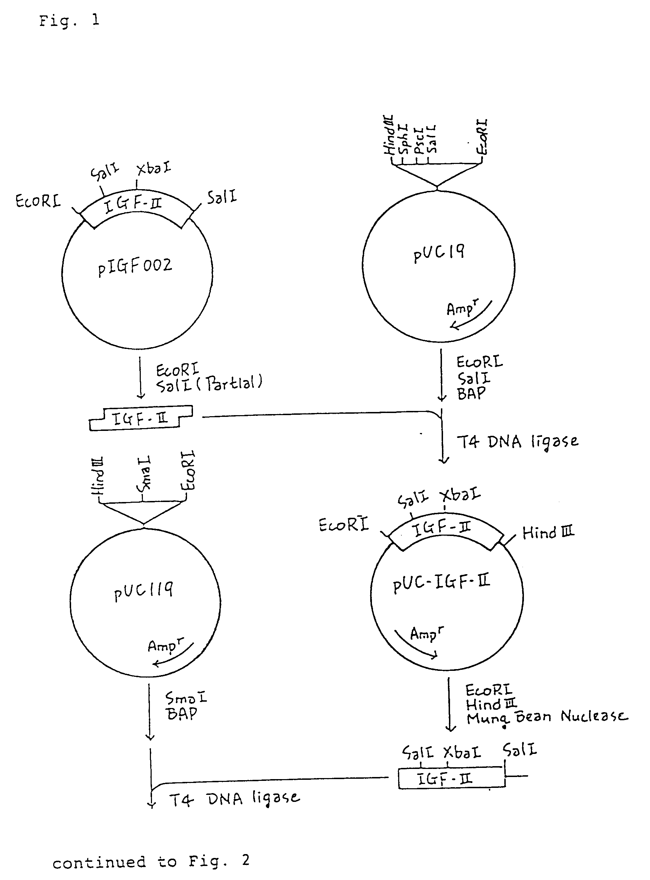

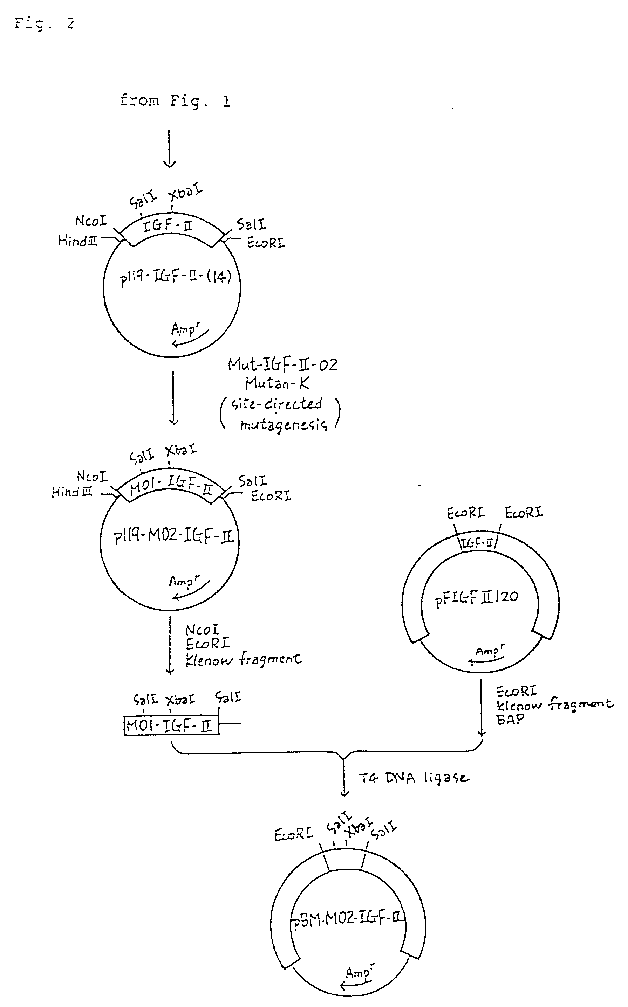

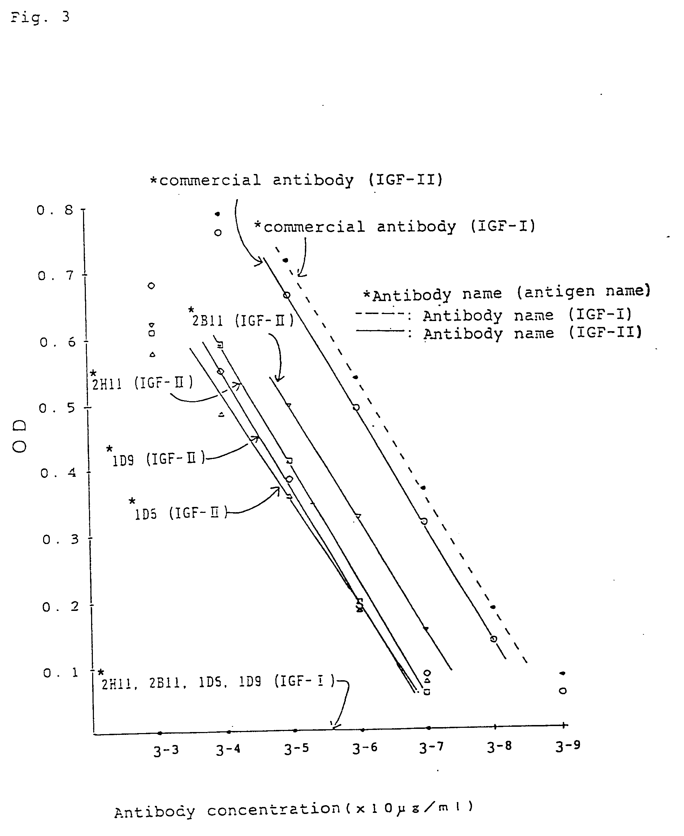

The processes involved from pIGF002 to pBM-M02-IGF-II for obtaining recombinant virus for expression are shown in Figs. 1 and 2.

(5) Preparation of Recombinant Virus vIGF-II-M02 for Expression:

Composition I shown below was prepared from DNA of silkworm nuclear polyhederosis virus BmNPV T3 (virus DNA of BmNPV T3 strain was deposited with American Type Culture Collection (address; 12301 Parklawn Drive, Rockville, MD 20852 USA) on June 13, 1985 under ATCC No. 40188) and pBM-M02-IGF-II at a molar ratio of 1:100. Composition I was mixed with composition II shown below.

| Composition I: | |
|---|---|
| Distilled water | 2.1 ml |
| Carrier DNA (salmon sperm, 1 mg/ml) | 50 $\mu$l |
| BmNPV DNA (1 mg/ml) | 10 $\mu$l |
| pBM-M02-IGF-II DNA | 50 $\mu$g |
| 2.0 M calcium chloride solution | 300 $\mu$g |

| Composition II: | |
|---|---|
| 50 mM HEPES buffer (pH = 7.1) containing 0.28 M sodium chloride | 2.5 ml |
| Phosphate buffer (3.5 mM $Na_2HPO_4$ -35 mM $NaH_2PO_4$) | 50 $\mu$l |

An 1 ml aliquot of the resulting cell suspension was added to 4 ml of a culture medium (TC-10 medium containing 10% fetal calf serum; see J. Invertebr. Pathol., Vol. 25, pp. 363-370 (1975)) for Bm cells (Bombyxmori cell line; deposited as BM cell (BM-N cell) with American Type Culture Collection (address; 12301 Parklawn Drive, Rockville, MD 20852 USA) on June 17, 1985 under ATCC No. CRL8910) to introduce the above-described DNA into the Bm cells. After 20 hours, the culture medium was exchanged with a fresh medium. After additional culturing for 5 days, the medium was collected and centrifuged to obtain the supernatant, from which cloned recombinant virus (designated vIGF-II-M02) was recovered by plaque assay (see J. Seric. Sci. Jpn., Vol. 53, pp. 547-548 (1984)).

(6) Expression of Polyhedron-Polypeptide-2 Fused Protein in Silkworm Larva:

A suspension of vIGF-II-M02 for expression (0.5 ml/insect; $10^7$ pfu) was percutaneously injected to 5-instar 1-day-old silkworms, and the silkworms were further fed and maintained at 25°C for 3 days to obtain polyhedron-polypeptide-2 fused protein expressing silkworms.

(7) Recovery of Polyhedron-Polypeptide-2 Fused Protein from Silkworm Bodies:

A hundred and twenty silkworms infected with vIGF-II-M02, and fed and grown for 3 days were soaked in 800 ml of water, and the bodies were completely homogenized in a high-speed homogenizer. The homogenate was subjected to ultrasonic wave treatment and filtered through double gauze. The filtrate and the washing of the gauze were combined, about 1200 ml in total, and centrifuged at 10,000 rpm for 20 minutes. The resulting precipitate was suspended in 250 ml of a 2.5 M urea solution and re-centrifuged under the same conditions as above to collect the precipitate. The precipitate was suspended in 150 ml of a 1.0 M guanidine hydrochloride solution and centrifuged under the same conditions to obtain an insoluble fraction containing polyhedron-polypeptide-2 fused protein. The resulting fraction was suspended in 360 ml of a 6.0 M guanidine hydrochloride solution and subjected to ultrasonic wave treatment while cooling with ice to dissolve the desired protein. To the solution was added 150 ml of ethyl ether, and the mixture was vigorously stirred and then treated with ultrasonic waves. The mixture was centrifuged in a Teflon-made centrifuge tube, whereby the system was separated into two phases having a deep green gel intermediate layer therebetween. The lower layer was collected and subjected to the same operations as described above two more times to thereby remove impurities. Then, the same operations were repeated, but using 150 ml of chloroform, and only the upper layer (guanidine solution) was collected. The guanidine solution was thoroughly dialyzed against 0.2% formic acid at 4°C. The thus formed white insoluble matter was removed by centrifugation at 12,000 rpm for 15 minutes. The supernatant liquid was lyophilized to obtain crude polyhedron-polypeptide-2 fused protein.

(8) Preparation of Polypeptide-2:

The crude polyhedron-polypeptide-2 fused protein obtained in (7) above was dissolved in 50 ml of 70% formic acid, followed by thoroughly degassing under reduced pressure. After the atmosphere was displaced with nitrogen gas, 200 mg of solid cyanogen bromide was added to the solution, and the system was sealed. The mixture was allowed to react at room temperature for 24 hours with stirring. The reaction mixture was diluted with about 15 times the volume of water, followed by lyophilization. The resulting powder was dissolved in 40 ml of 10 M urea, and the solution was adjusted to a urea concentration of 6.0 M by addition of a 20 mM acetic acid buffer (pH = 4.0) and then to a pH of 4.0 by addition of acetic acid. The resulting solution was applied to a column (2.5 cm (D) x 6.0 cm (H)) packed with SP-TOYOPEARL 650 M (produced by Toso Co., Ltd.) which had been sufficiently equilibrated with a 20 mM acetic acid buffer (pH = 4.0) containing 6.0 M urea to let the desired substance be adsorbed on to the adsorbent. The column was sufficiently washed with 150 ml of the same buffer as used above, and the adsorbate was eluted with 150 ml of the same buffer and 150 ml of NaCl-containing same buffer at an NaCl linear concentration gradient of from 0 to 0.4 M to take 16 ml-fractions in a fraction collector. After analysis by SDS-polyacrylamide gel electrophoresis to confirm the position, each fraction was recovered. The resulting solution was adjusted to a pH of about 8.0, and 2-mercaptoethanol was added thereto to a final concentration of 100 mM, followed by allowing the solution to stand at room temperature for 4 hours to conduct reduction. The solution was sufficiently dialyzed at 4°C using a membrane "SPECTRA/PORE 3" (produced by Spectrum Co., Ltd.) against 20 mM acetic acid buffer (pH = 4.0) containing 6.0 M urea and 50 mM 2-mercaptoethanol. The dialysate was applied to an SP-TOYOPEARL column (1.5 cm (D) x 6.0 cm (H)) previously equilibrated with the same buffer, and the column was treated in the same manner as in the above-described column chromatography (gradient elution from 0 to 0.4 M NaCl; in the presence of 50 mM 2-mercaptoethanol) to obtain partially purified reduced polypeptide fractions. The resulting solution was sufficiently dialyzed against 0.1% formic acid using the same membrane as used above, and the dialysate was lyophilized to obtain 220 mg of a powder. To the powder was added 30 ml of a 6.0 M guanidine hydrochloride solution, followed by stirring for 30 minutes under reduced pressure to dissolve the powder. To the solution was slowly added 150 ml of 50 mM Tris•HCl buffer (pH = 8.4) under stirring. The stirring was continued under reduced pressure for an additional period of about 1 hour and, immediately thereafter, the container was sealed by covering the opening with a polymer film (PARAFILM). Several holes were made through the film cover with a needle so as to let air gradually enter into the container, and the container was allowed to stand at room temperature for 3 days to carry out air oxidation. The reaction mixture was concentrated to about 25 ml by ultrafiltration using "Diaflow Membrane YM-5" (produced by Amicon Co., Ltd.). Final purification of the oxidized polypeptide-2 was carried out by HPLC for fractionation using a chromatograph "LC-8A" (produced by Shimadzu Co., Ltd.) under the following conditions.

HPLC-1:

| Column: | CAPCELL PAK $C_{18}$ (SG type 5 $\mu$m; 1.5 cm (D) x 25 cm (H); manufactured by Shiseido Co., Ltd.) |
|---|---|
| Flow rate: | 8.0 ml/min |
| Elution: | gradient elution with 0.1% trifluoroacetic acid containing from 20% up to 55% acetonitrile (50 minutes) |
| Detection: | UV absorption at 210 nm |

Chromatography was repeated three times, and the desired fractions collected for a retention time of 24.0 minutes were combined and lyophilized. The resulting powder was again applied to HPLC using the same column for final purification under the following conditions.

HPLC-2:

| Column, flow rate, and detection conditions: | the same as in HPLC-1 |
|---|---|
| Elution: | gradient elution with 0.1% trifluoroacetic acid containing from 26% up to 34% acetonitrile (40 minutes) |

Chromatography was repeated twice, and the main peak fractions for a retention time of about 21.5 minutes were collected, combined, and lyophilized to obtain about 10 mg of polypeptide-2 powder.

EXAMPLE 2: Preparation of Monoclonal Antibody

(1) Preparation of Antigen Solution:

To a solution of 3 mg of polypeptide-2 in 0.5 ml of 0.4% SDS/0.1 M phosphate buffer (pH = 7.4) was added a solution of 2.5 mg of bovine serum albumin (BSA) in 0.5 ml of 0.1 M phosphate buffer (pH = 7.4), and 0.5 ml of 20 mM glutaraldehyde was further added to the mixture, followed by stirring at room temperature for 30 minutes. The reaction mixture was thoroughly dialyzed against phosphate-buffered saline, and the dialysate was centrifuged to obtain the supernatant, peptide-2/BSA conjugate.

(2) Immunization:

Peptide-2/BSA conjugate (0.3 mg) was thoroughly emulsified with the equal volume of Freund's complete adjuvant (FCA; produced by Difco Laboratories), and the emulsion was subcutaneously administered to three female BALB/c mice (7- to 8-week-old; purchased from Japan Charles River Co., Ltd.) at a dose of 100 $\mu$g-antibody/mouse. Separately, 0.3 mg of IGF-II was thoroughly emulsified with the equal volume of Freund's incomplete adjuvant (FICA; produced by Difco Laboratories). After 3 weeks from the primary immunization, the IGF-II emulsion was subcutaneously administered to the mice at a booster dose of 0.1 mg-antibody/mouse. The booster was repeatedly conducted every 2 weeks, and the blood was sampled 7 days after each booster to determine antibody titer of the serum. For final immunization, 2 mice showing a high antibody titer were chosen, and 0.5 ml of physiological saline containing 0.1 mg of peptide-2/BSA conjugate was intraperitoneally administered. Three days after the final immunization, the spleen was excised for use in the subsequent cell fusion.

(3) Cell Fusion:

The spleen was sterilely excised from each of the two mice and washed with serum-free PRMI 1640 (produced by Nissui Pharmaceutical Co., Ltd.). A few cuts were made in the spleen, and the spleen cells were pressed out with the frosted part of slide glass. The spleen cells were washed with a Tris•$NH_4$Cl solution to remove erythrocytes to prepare spleen cells to be used for cell fusion. The resulting spleen cells and mouse myeloma cells P3 x63 Ag8 U1 were mixed at a ratio of 5:1 (cell number). After thorough removal of the medium, the mixed cells were incubated in 1 ml of 50% polyethylene glycol 1500 at 37°C for 2 minutes to perform cell fusion. The resulting cell mixture was washed with serum-free PRMI medium and then suspended in HAT (1 x $10^{-4}$ M hypoxanthine, 4 x $10^{-7}$ M aminopterin, 1.6 x $10^{-5}$ M thymidine)-added 10% fetal calf serum/RPMI 1640 medium, and $10^5$ cells were pored to each well. The cells were cultured at 37°C under an atmosphere of 7% $CO_2$. The antibody titer of the resulting hybridoma population containing hybridoma 2H11, 2B11, ID5, and ID9 was determined as follows to choose wells to be subjected to cloning.

(4) Determination of Antibody Titer:

Determination of antibody titer during mouse immunization and screening were carried out according to enzyme-labeled antibody technique (ELISA) as follows.

To each well of a 96-well microplate (produced by Biotec Co., Ltd.) was added 0.05 ml of 25 mM carbonic acid buffer (pH = 9.0) containing 10 $\mu$g/ml of IGF-II, followed by allowing the reaction mixture to stand at 4°C overnight to allow the antigen to be adsorbed. Each well was washed three times with 0.05% Tween 20/PBS, and 0.1 ml of 0.5% BSA/0.05% Tween 20/PBS was added thereto, followed by allowing the reaction mixture to stand at 37°C for 3 hours. The well was washed three times with 0.05% Tween 20/PBS, and 0.05 ml/well of the antiserum of the mouse or its culture supernatant which was continuously and stepwise diluted with 0.5% PBS/0.05% Tween 20/PBS, and the system was allowed to stand at 29°C for 90 minutes to conduct an antigen-antibody reaction. After washing each well three times with 0.05% Tween 20/PBS, 0.05 ml/well of horseradish peroxidase (HRP)labeled anti-mouse IgG goat antibody (produced by Funakoshi Co., Ltd.) which was 2000-fold diluted with 0.5% BSA/0.05% Tween 20/PBS was added thereto, followed by allowing the reaction mixture to stand at 29°C for 1 hour. After washing each well three times with 0.05% Tween 20/PBS, 0.05 ml/well of a substrate solution (ABTS, ZYMED) was added thereto, and colorimetric analysis was conducted by absorbance at 405 nm.

(5) Selection of Antibody-Producing Cells:

The antibody titer of the hybridoma population obtained in (3) above was determined by ELISA on the culture supernatant after 7 days from the cell fusion. Primary cloning of the cells in each of the wells which were observed to have produced the antibody was conducted by limiting dilution method. Then, a monoclonal hybridoma was obtained by secondary cloning. The thus obtained cells were designated hybridoma 2H11, 2B11, ID5 and ID9.

Hybridoma 2H11 producing monoclonal antibody 2H11, hybridoma 2B11 producing monoclonal antibody 2B11, hybridoma ID5 producing monoclonal antibody ID5, and hybridoma ID9 producing monoclonal antibody ID9 were deposited with Fermentation Research Institute, Agency of Industrial Science & Technology (address; 1-3, Higashi 1 chome, Tsukuba-shi, Ibaraki-ken 305, Japan) on February 14, 1991, MITI under receipt numbers FERM BP-3275, FERM BP-3274, FERM BP-3276, and FERM BP-3277, respectively.

EXAMPLE 3: Determination of Class and Subclass of Monoclonal Antibody

The subclass of the monoclonal antibodies produced by the hybridoma cells obtained in Example 2 was determined by ELISA. IGF-II was immobilized on a plate for ELISA and reacted with the culture supernatant of each hybridoma and further reacted with an HRP-labeled antibody against mouse immunoglobulin of each class and subclass (IgG(Fc), IgGl, IgG2a, IgG2b, IgG3, IgA and IgM). As a negative control sample, the culture supernatant of myeloma P3 x63 Ag8 U1 was also analyzed, and those samples showing absorbance twice or more that of the negative control sample were judged positive. The results obtained are shown in Table 1 below.

As a result, each of the resulting monoclonal antibodies were found to belong to the IgG1 class.

TABLE 1

| Determination of Class and Subclass of Monoclonal Antibody | | | | |
|---|---|---|---|---|
| Secondary Antibody | Absorbance Ratio and Judgement | | | |
| | 2H11 | 2B11 | ID5 | ID9 |
| IgG (Fc) | 2.4 + | 8.4 + | 9.5 + | 7.5 + |
| IgG1 | 5.0 + | 18.0 + | 15.3 + | 13.1 + |
| IgG2a | 0.9- | 0.9- | 1.0- | 1.0- |
| IgG2b | 1.0- | 1.1- | 1.1- | 1.1- |
| IgG3 | 1.0- | 1.3- | 1.0- | 1.0- |
| IgA | 0.9- | 1.1- | 1.0- | 1.0- |
| IgM | 1.1- | 0.9- | 0.9- | 0.9- |

EXAMPLE 4: Preparation and Purification of Ascitic Monoclonal Antibody

For the purpose of obtaining ascitic monoclonal antibodies, 0.5 ml/animal of pristan (2,6,10,14-tetramethylpentadecane, produced by Wako Pure Chemical Industries, Ltd.) was intraperitoneally administered to 15-to 20-week-old BALB/C mice (purchased from Japan Charles River Co., Ltd.). About 3 weeks from the administration, $5 \times 10^6$ cells/animal of hybridoma 2H11, 2B11, ID5, or ID9 was intraperitoneally implanted. Two to three weeks later, the ascites was collected.

The ascites was purified by affinity chromatography using a Protein A column "PROSEP-A 'HIGH CAPACITY'" (produced by Bioprocessing Co., Ltd.) as follows. The ascites (20 ml) was diluted with a double volume of 1.0 M glycine•NaOH/0.15 M NaCl (pH = 8.0) and applied to a PROSEP-A column (1.0 (D) x 7.5 cm (H)), the column was washed with about 70 ml of 0.1 M glycine•NaOH/0.15 M NaCl, and the antibody absorbed was eluted with 0.1 M citric acid buffer (pH = 3.5). Each eluate was concentrated by ultrafiltration using "YM 30" (produced by Amicon Co.) as a membrane, and the concentrate was thoroughly dialyzed against PBS at 4°C.

The following Examples were carried out by using the thus obtained ascitic monoclonal antibody 2H11, 2B11, ID5, or ID9.

EXAMPLE 5: Reaction Specificity of Monoclonal Antibody to IGF-II

The procedures of ELISA described in Example 2 were as follows. Each of the purified monoclonal antibodies obtained in Example 4 was stepwise diluted starting from a concentration of 10 μg/ml by 3-fold for each dilution and reacted with an immobilized antigen, recombinant IGF-II (J. gen. Virol., Vol. 68, pp. 2599-2606 (1987)) or recombinant IGF-I (produced by TOYOBO Co., Ltd.). The antigen-antibody complex was further reacted with HRP-labeled anti-mouse IgG goat antibody, and the HRP activity of each well was determined. As a positive control, a commercially available anti-IGF-II monoclonal antibody known to have cross reactivity to IGF-I (produced by Amano Pharmaceutical Co., Ltd.) was used. The 96-well microplate used here was that manufactured by Coster Co.

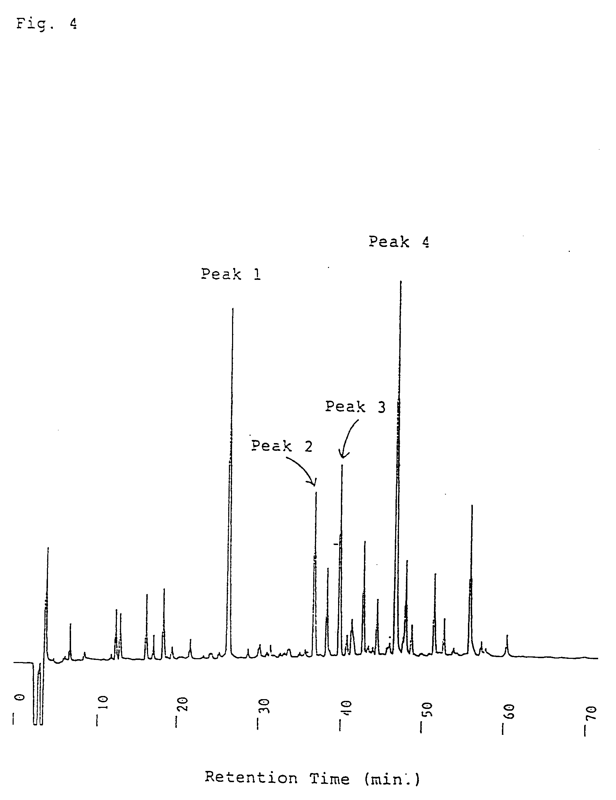

The results obtained are shown in Fig. 3.

It is seen that monoclonal antibody 2H11, 2B11, ID5 and ID9 react with IGF-II but do not react at all with IGF-I, while the commercially available anti-IGF-II monoclonal antibody reacts with both IGF-II and IGF-I.

EXAMPLE 6: Determination of Epitope

(1) Preparation of Peptide Fragment of IGF-II Origin by Pepsin Digestion:

Digestion of IGF-II by pepsin was conducted by substantially following the method of Smith, et al. (see J. Biol. Chem., Vol. 264, pp. 9314-9321 (1989)). In 250 μl of 0.01 N HCl was dissolved 100 μg of IGF-II powder, and 5 μg of pepsin (produced by Sigma Co.) was added to the solution. The mixture was allowed to react at room temperature for 48 hours. The peptide fragments in the reaction mixture were isolated and purified by HPLC under the following conditions, and an eluate containing each peptide fragment was lyophilized.

Column: CAPCELL PAK C$_{18}$ SG 120 (4.6 mm (D) x 250 mm (H), produced by Shiseido Co., Ltd.)
Solvent A: 0.1% trifluoroacetic acid-containing water
Solvent B: 0.1% trifluoroacetic acid-containing acetonitrile
Flow rate: 1.00 ml/min
Detection: UV 210 nm
Elution: linear gradient from 5% to 35% acetonitrile in 0.1% trifluoroacetic acid (90 minutes)

The elution pattern is shown in Fig. 4. Peaks 1 to 4 in the figure were used for determination of the epitope for each monoclonal antibody. Assignment of each peptide fragment corresponding to each peak to an amino acid sequence of IGF-II was carried out by amino acid sequence analysis using "Protein Sequencer 470A" (manufactured by Applied Biosystem Co.) and amino acid composition analysis.

The amino acid sequence of each peptide fragment is shown below.

```
Peak 1:        Phe-Ser-Arg-Pro-Ala-Ser-Arg-Val-Ser-Arg-Arg-Ser-Arg-Gly (SEQ ID NO:1)

Peak 2:        Ala-Tyr-Arg-Pro-Ser-Glu-Thr-Leu (SEQ ID NO:4)

Peak 3:               Cys-Gly-Gly-Glu-Leu
                       |
               Glu-Cys-Cys-Phe-Arg-Ser-Cys-Asp
                       |_____|


Peak 4:        Phe-Val-Cys-Gly-Asp-Arg-Gly-Phe-Tyr
                       |
               Tyr-Cys-Ala-Thr-Pro-Ala-Lys-Ser-Glu
```

(2) ELISA Using Each Peptide Fragment as Antigen:

Procedures of ELISA in Example 3 were substantially followed. Each peptide fragment was immobilized on a 96-well microplate, and 1.1 μg/ml/well of antibody 2H11, 2B11, ID5, or ID9 was added to conduct an antigen-antibody reaction. The antigen-antibody complex was further reacted with HRP-labeled anti-mouse IgG goat antibody, and the HRP activity of each well was determined. The results obtained are shown in Table 2 below in terms of optical density (OD). For reference, the same analysis was conducted using a commercially available anti-IGF-II monoclonal antibody (produced by Amano Pharmaceutical Co., Ltd.) as an antibody, and IGF-II and IGF-I as an antigen. The results obtained are also shown in Table 2.

The results in Table 2 indicate that the epitope of IGF-II recognized by monoclonal antibody 2H11 exist in the partial amino acid sequence of from the 28th Phe to the 41st Gly. It is also seen that each of the monoclonal antibodies obtained in the present invention reacts with IGF-II but not with IGF-I, while the commercially available anti-IGF-II monoclonal antibody (referred to as AMANO in Table 2) reacts with both IGF-II and IGF-I.

TABLE 2

| Epitope Determination | | | | | |
|---|---|---|---|---|---|
| Antibody | Peptide Fragment (OD) | | | | Reference Example | |
| | Peak 1 | Peak 2 | Peak 3 | Peak 4 | IGF-II | IGF-I |
| 2H11 | 0.821 | 0.002 | 0.003 | 0.000 | 1.373 | 0.005 |
| 2B11 | 0.002 | -0.001 | 0.002 | 0.000 | 1.237 | 0.003 |
| ID5 | -0.001 | 0.003 | 0.003 | 0.002 | 1.142 | 0.005 |
| ID9 | 0.000 | 0.003 | -0.001 | -0.001 | 1.191 | 0.003 |
| AMANO | 0.000 | 0.008 | 0.007 | 0.010 | 1.296 | 1.344 |

EXAMPLE 7: Specific Determination of IGF-II

(1) Sandwich ELISA (J. Biochem., Vol. 92, pp. 1413-1424 (1982)):

Anti-IGF monoclonal antibody ID9 (6.25 mg) was thoroughly dialyzed against 10 mM carbonic acid buffer (pH = 9.5). Separately, 4.0 mg of HRP (produced by Boehringer Mannheim Co.) was dissolved in 1.0 ml of water, and 0.2 ml of 0.1 M sodium periodate solution was added thereto to conduct a reaction at room temperature for 20 minutes. The reaction mixture was dialyzed against 1 mM acetic acid buffer (pH = 4.4) at 4°C overnight. The dialysate (1.40 ml) was adjusted to a pH of about 9 by addition of 0.2 M sodium carbonate solution, and a 1.05 ml aliquot of the solution was added to the above-prepared dialysate of the antibody, followed by stirring at room temperature for 2 hours. To the reaction mixture was added 5 mg of sodium cyanoborohydride, followed by allowing the reaction mixture to stand at room temperature for 2 hours to reduce the Schiff base produced by the reaction. Then, 1.0 ml of 0.2 M Tris•HCl/0.1 M monoethanolamine was added thereto, followed by allowing the reaction mixture to stand at room temperature for 90 minutes. The reaction mixture was sufficiently dialyzed against PBS at 4°C to obtain an HRP-labeled antibody solution.

IGF-II determination was conducted in substantially the same manner as in Example 3. An anti-IGF-II antibody 2B11 was immobilized on a 96-well microplate (produced by Coster Co.) and reacted with IGF-II, IGF-I (produced by TOYOBO Co., Ltd.) or insulin (produced by Sigma Co.) in various concentrations. The resulting antigen-antibody complex was further reacted with the above-prepared HRP-labeled anti-IGF-II antibody ID9, and the HRP activity of each well was assayed.

The results obtained are shown in Table 3 below. The monoclonal antibodies of the present invention specifically reacted with IGF-II, making it possible to quantitatively determine IGF-II.

TABLE 3

| Antigen | Concentration (ng/ml) | Absorbance (405 nm) |
|---|---|---|
| IGF-II (n = 4) | 100 | 0.473 |
| | 50 | 0.247 |
| | 25 | 0.103 |
| | 12.5 | 0.041 |
| IGF-I (n = 4) | 2000 | 0.009 |
| | 1000 | -0.010 |
| Insulin (n = 4) | 5000 | 0.000 |
| | 2500 | 0.000 |

According to the present invention, a plurality of highly specific anti-human IGF-II monoclonal antibodies differing in recognition site are obtained. A combination of these antibodies makes an ELISA feasible, which leads to precise determination of human IGF-II.

(2) Competition ELISA:

Preparation of HRP-labeled IGF-II was carried out according to the maleimide method described previously (J. Biochem., Vol. 92, pp. 1413-1424 (1982)).

Horseradish peroxidase (3.3 mg) was dissolved in 0.3 ml of 0.1 M sodium phosphate buffer (pH 7.0), and incubated with N-hydroxysuccinimide ester of N-(4-carboxy-cyclohexylmethyl)-maleimide(0.75 mg) in 75 $\mu$l of N,N-dimethylformamide at 30°C for 1 hour with continuous stirring. Precipitate formed was removed by centrifugation and the supernatant was subjected to gel filtration on a column (1.0 cm (D) x 38 cm (H)) of Sephadex G-25 using 0.1 M sodium phosphate buffer (pH 6.0). Fractions containing desired protein were pooled and concentrated by ultrafiltration using "DIAFLOW MEMBRANE YM-30" (produced by Amicon Co., Ltd.) as a membrane to obtain maleimide-HRP (0.2 ml).

In order to conjugate above maleimide-HRP and IGF-II, thiol groups were generated to IGF-II as followed. IGF-II (0.8 mg) was dissolved in 0.6 ml of 0.1 M sodium phosphate buffer (pH 6.5), and S-acetylmercaptosuccinic anhydride (2.0 mg) in 35 $\mu$l of N,N-dimethylformamide was added, followed by allowing the mixture to incubate at 30°C for 30 minutes. To this solution, 40 $\mu$l of 0.1 M EDTA, 200 $\mu$l of 0.1 M Tris•HCl (pH 7.0) and 200 $\mu$l of 1.0 M hydroxylamine/HCl were added, followed by allowing the mixture to stand at room temperature for 4 minutes. The reaction mixture was subjected to gel filtration on a column (1.0 cm (D) x 41 cm (H)) of Sephadex G-25 using 0.1 M sodium phosphate buffer (pH 6.0) containing 5 mM EDTA. Fractions containing desired protein were collected and concentrated to 0.5 ml by ultrafiltration using "DIAFLOW MEMBRANE YM-5" (produced by Amicon Co., Ltd.).

To this solution, above maleimide-HRP was mixed. After incubation at 4°C for 20 minutes, 70 $\mu$l of 0.1 M N-ethylmaleimide was added to this reaction mixture, then subjected to gel filtration on the same column using 0.1 M sodium phosphate buffer (pH 6.5). Fractions containing desired protein were collected and were concentrated by ultrafiltration using "DIAFLOW MEMBRANE YM-30" (produced by Amicon Co., Ltd.). After concentrating to about 1 ml, inner solution was diluted 10-folds with PBS, and then continuously concentrated. These procedures were repeated 3 times to obtain HRP-labeled IGF-II (1.1 ml).

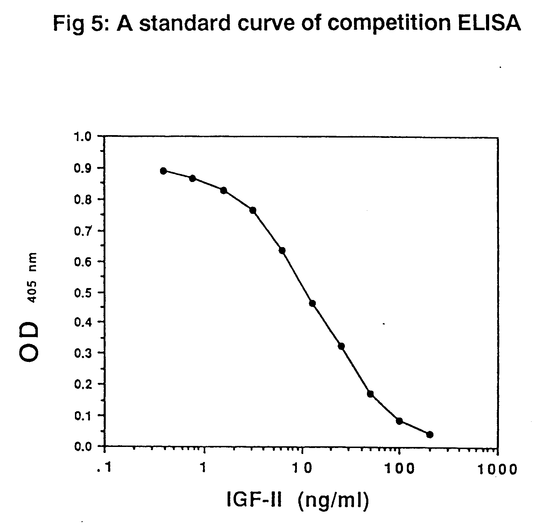

To each well of a 96-well microplate (produced by Coster Co.) was added 0.05 ml of PBS containing a mixture of each 2.5 $\mu$g/ml of four antibodies (2H11, 2B11, ID5 and ID9), followed by allowing the mixture to stand at 4°C overnight. Each well was washed with 0.05% Tween 20/PBS, and 0.1 ml of 0.05% BSA/0.02% Tween 20/PBS was added thereto, followed by allowing the mixture to stand at 37°C for 3 hours. After washing 3 times with 0.05% Tween 20/PBS, 0.025 ml of the above-prepared HRP-labeled IGF-II which was 100,000-folds diluted with 0.1% BSA/0.02% Tween 20/PBS was added to each well, and then 0.025 ml of various concentration of IGF-II was added thereto. After incubation at 4°C overnight, each well was washed 3 times with 0.05% Tween 20/PBS, and the HRP activity of each well was assayed. The results obtained are shown in Fig. 5.

According to the present invention, a highly specific anti-human IGF-II monoclonal antibodies are obtained. These antibodies make an ELISA feasible, which leads to precise determination of human IGF-II.

EXAMPLE 8: Western Blotting Analysis

12

EP 0 492 552 A1

(1) Preparation of Anti-IGF-II Antibody (2H11)-Conjugated Resin:

Anti-IGF-II monoclonal antibody 2H11 (4.5 mg) was dissolved in 5 ml of 0.25 M sodium phosphate buffer (pH 8.0). To this solution was added 4 ml of FORMYL-CELLULOFINE (produced by Seikagaku Kogyo Co., Ltd.) which had been sufficiently washed with 100 ml of water, and then stirred at room temperature for 2 hours. Then, 10 mg of powdered sodium cyanoborohydride was added to this solution, and further stirred at room temperature for 3 hours. After filtration, antibody-conjugated resin was washed with 100 ml of water. The resulting resin was suspended in 10 ml of 0.2 M Tris•HCl buffer (pH 7.2) containing 0.1 M monoethanolamine, and 10 mg of powdered sodium cyanoborohydride was added, followed by allowing the mixture to stand at room temperature for 3 hours. After filtration, resin was washed with 100 ml of water to give desired antibody 2H11-conjugated resin.

(2) Preparation of Serum-Derived Sample for Western Blotting Analysis:

A 300 $\mu$l aliquot of serum derived from a normal adult or a patient with gastric cancer and severe hypoglycemia was mixed with 1,200 $\mu$l of 87.5% ethanol-2.0 N HCl in a polypropylene tube. The mixture was incubated at room temperature for 45 minutes and then centrifuged at 15,000 rpm for 10 minutes at 4°C. A 1.2 ml aliquot of the supernatant was neutralized in a separate tube by adding 2 M ammonium bicarbonate. Ethanol was removed in vacuo, followed by lyophilization. Lyophilized powder was dissolved in 1 ml of PBS containing 0.02% Tween 20. To this solution was added above antibody 2H11-conjugated resin (50 $\mu$l), and then incubated on vortex mixer at room temperature for 3 hours. IGF-II-adsorbed resin was washed once with PBS containing 0.04% Tween 20 and 1M NaCl, and then with water. Then, the resin was treated with 100 $\mu$l of Laemmli sample buffer (Nature, Vol. 227, pp. 680-685 (1970)) to solubilize and recover IGF-II from antibody-antigen complex on the resin. The desired serum-derived IGF-II was contained in supernatant.

(3) Preparation of IGF-II-Producing Tumor Tissue-Derived Sample for Western Blotting Analysis:

The lump of IGF-II-producing human tumor tissue (wet weight, 156 mg) was homogenized in 1.5 ml of 70% ethanol-2.0 N HCl in a polypropylene tube using "POLYTRON" homogenizer (purchased from KINEMATICA GmbH LITTAU). The homogenate was incubated at room temperature for 45 minutes and then centrifuged at 15,000 rpm for 10 minutes at 4°C. Finally, the desired tumor tissue-derived IGF-II was obtained by the same procedure described above.

(4) Sodium Dodecyl Sulfate-Polyacrylamide Gel Electrophoresis, Membrane Transfer and Detection:

Aliquots of authentic IGF-II, IGF-I, serum-derived IGF-II and tumor tissue-derived IGF-II were subjected to electrophoresis using the sodium dodecyl sulfate-polyacrylamide gel electrophoresis (hereinafter abbreviate SDS-PAGE) system of Laemmli (Nature, Vol. 227, pp. 680-685 (1970)). The SDS-PAGE was carried out at a constant 25 milliamperes using a 16% polyacrylamide slab gel without 2-mercaptoethanol. After electrophoresis, the proteins on gel were transferred to PVDF membrane ("Pro Blott", produced by Applied Biosystems) in 10 mM CAPS buffer (pH 11) containing 10% methanol for 1 hour with a constant 50 volts using "Mini Trans-Blot" apparatus (purchased from Bio Rad Lab.). Non-specific binding sites on the membrane were blocked by immersing the membrane in PBS containing 5% skim milk and 0.05% Tween 20 for 16 hours at 4°C on a reciprocal incubator. The membrane was treated first with PBS containing 0.05% Tween 20 and 1 $\mu$g/ml of antibody 2H11, and treated with the same buffer containing 0.05% Tween 20 and 5,000-folds diluted HRP-conjugated anti-mouse IgG-sheep IgG (Amersham Corp.) at room temperature for 1 hour. Detection of IGF-IIs on the membrane was carried out using ECL Western Blotting Detection Kit (Amersham Corp.) according to its manual.

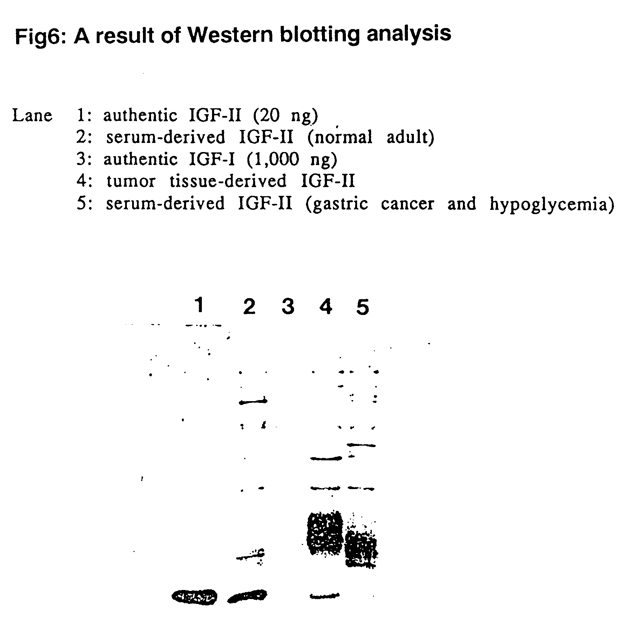

The results obtained are shown in Fig. 6. Authentic IGF-II, serum-derived IGF-II and tumor tissue-derived IGF-II were fully detectable, but excess amount of IGF-I could not be detected. Furthermore, these results indicate that this system are effective to detect and analyze not only the normal form of IGF-II (7.5 kD) but also higher molecular forms of IGF-II whose presence were reported in the case of patients with tumor-induced hypoglycemia. (Proc. Natl. Acad. Sci., USA, Vol. 82, pp. 3169-3172 (1985)).

Useful antibodies of the present invention can also be made use of as a diagnostic reagent for determining human IGF-II blood level, or in various analysis of human IGF-II, such as Western blotting analysis, or for purification of human IGF-II.

While the invention has been described in detail and with reference to specific examples thereof, it will

13

be apparent to one skilled in the art that various changes and modifications can be made therein without departing from the spirit and scope thereof.

## Claims

1. A monoclonal antibody that specifically binds to human insulin-like growth factor II (IGF-II) but does not bind to human insulin-like growth factor I (IGF-I).

2. A monoclonal antibody as claimed in Claim 1, that specifically recognize an amino acid sequence of Phe-Ser-Arg-Pro-Ala-Ser-Arg-Val-Ser-Arg-Arg-Ser-Arg-Gly (SEQ ID NO:1).

3. A monoclonal antibody as claimed in Claim 1, that does not recognize an amino acid sequence of Phe-Ser-Arg-Pro-Ala-Ser-Arg-Val-Ser-Arg-Arg-Ser-Arg-Gly (SEQ ID NO:1).

4. A process for preparing a monoclonal antibody belonging to the immunoglobulin IgG class that specifically binds to human insulin-like growth factor II (IGF-II) but does not bind to human insulin-like growth factor I (IGF-I), comprising immunizing a mammal using, as an antigen, a polypeptide having an amino acid sequence of Ala-Tyr-Arg-Pro-Ser-Glu-Thr-Leu-Cys-Gly-Gly-Glu-Leu-Val-Asp-Thr-Leu-Gln-Phe-Val-Cys-Gly-Asp-Arg-Gly-Phe-Tyr-Phe-Ser-Arg-Pro-Ala-Ser-Arg-Val-Ser-Arg-Arg-Ser-Arg-Gly-Ile-Leu-Glu-Glu-Cys-Cys-Phe-Arg-Ser-Cys-Asp-Leu-Ala-Leu-Leu-Glu-Thr-Tyr-Cys-Ala-Thr-Pro-Ala-Lys-Ser-Glu (SEQ ID NO:2), preparing hybridoma cells from antibody-producing cells of the immunized mammal and myeloma cells, selecting a desired hybridoma, culturing the hybridoma, and collecting the antibody.

5. A monoclonal antibody that specifically binds to human insulin-like growth factor II (IGF-II) but does not bind to human insulin-like growth factor I (IGF-I) and which is produced by a process comprising immunizing a mammal using, as an antigen, a polypeptide having an amino acid sequence of Ala-Tyr-Arg-Pro-Ser-Glu-Thr-Leu-Cys-Gly-Gly-Glu-Leu-Val-Asp-Thr-Leu-Gln-Phe-Val-Cys-Gly-Asp-Arg-Gly-Phe-Tyr-Phe-Ser-Arg-Pro-Ala-Ser-Arg-Val-Ser-Arg-Arg-Ser-Arg-Gly-Ile-Leu-Glu-Glu-Cys-Cys-Phe-Arg-Ser-Cys-Asp-Leu-Ala-Leu-Leu-Glu-Thr-Tyr-Cys-Ala-Thr-Pro-Ala-Lys-Ser-Glu (SEQ ID NO:2).

6. A hybridoma which produces a monoclonal antibody as claimed in Claim 1, which is produced by a process comprising immunizing a mammal using, as an antigen, a polypeptide having an amino acid sequence of Ala-Tyr-Arg-Pro-Ser-Glu-Thr-Leu-Cys-Gly-Gly-Glu-Leu-Val-Asp-Thr-Leu-Gln-Phe-Val-Cys-Gly-Asp-Arg-Gly-Phe-Tyr-Phe-Ser-Arg-Pro-Ala-Ser-Arg-Val-Ser-Arg-Arg-Ser-Arg-Gly-Ile-Leu-Glu-Glu-Cys-Cys-Phe-Arg-Ser-Cys-Asp-Leu-Ala-Leu-Leu-Glu-Thr-Tyr-Cys-Ala-Thr-Pro-Ala-Lys-Ser-Glu (SEQ ID NO:2), and preparing hybridoma cells from antibody-producing cells of the immunized mammal and myeloma cells, followed by cloning.

7. A hybridoma which produces a monoclonal antibody as claimed in Claim 2, which is produced by a process comprising immunizing a mammal using, as an antigen, a polypeptide having an amino acid sequence of Ala-Tyr-Arg-Pro-Ser-Glu-Thr-Leu-Cys-Gly-Gly-Glu-Leu-Val-Asp-Thr-Leu-Gln-Phe-Val-Cys-Gly-Asp-Arg-Gly-Phe-Tyr-Phe-Ser-Arg-Pro-Ala-Ser-Arg-Val-Ser-Arg-Arg-Ser-Arg-Gly-Ile-Leu-Glu-Glu-Cys-Cys-Phe-Arg-Ser-Cys-Asp-Leu-Ala-Leu-Leu-Glu-Thr-Tyr-Cys-Ala-Thr-Pro-Ala-Lys-Ser-Glu (SEQ ID NO:2), and preparing hybridoma cells from antibody-producing cells of the immunized mammal and myeloma cells, followed by cloning.

**8.** A hybridoma as claimed in Claim 7, wherein said hybridoma is hybridoma 2H11 having receipt number of FERM BP-3275.

**9.** A hybridoma which produces a monoclonal antibody as claimed in Claim 3, which is produced by a process comprising immunizing a mammal using, as an antigen, a polypeptide having an amino acid sequence of Ala-Tyr-Arg-Pro-Ser-Glu-Thr-Leu-Cys-Gly-Gly-Glu-Leu-Val-Asp-Thr-Leu-Gln-Phe-Val-Cys-Gly-Asp-Arg-Gly-Phe-Tyr-Phe-Ser-Arg-Pro-Ala-Ser-Arg-Val-Ser-Arg-Arg-Ser-Arg-Gly-Ile-Leu-Glu-Glu-Cys-Cys-Phe-Arg-Ser-Cys-Asp-Leu-Ala-Leu-Leu-Glu-Thr-Tyr-Cys-Ala-Thr-Pro-Ala-Lys-Ser-Glu (SEQ ID NO:2), and preparing hybridoma cells from antibody-producing cells of the immunized mammal and myeloma cells, followed by cloning.

**10.** A hybridoma as claimed in Claim 9, which is selected from the group consisting of hybridoma 2B11 having receipt number of FERM BP-3274, hybridoma ID5 having receipt number of FERM BP-3276, and hybridoma ID9 having receipt number of FERM BP-3277.

**11.** Hybridoma 2B11 having receipt number of FERM BP-3274.

**12.** Hybridoma ID5 having receipt number of FERM BP-3276.

**13.** Hybridoma ID9 having receipt number of FERM BP-3277.

**14.** A immunochemical method for specific detection of human insulin-like growth factor-II (IGF-II) using a monoclonal antibody as claimed in Claim 1.

**15.** A immunochemical method for specific detection of human insulin-like growth factor-II (IGF-II) using a monoclonal antibody produced by a hybridoma which is selected from the group consisting of hybridoma 2H11 having receipt number of FERM BP-3275, hybridoma 2B11 having receipt number of FERM BP-3274, hybridoma ID5 having receipt number of FERM BP-3276, and hybridoma ID9 having receipt number of FERM BP-3277.

Fig. 1

continued to Fig. 2

Fig. 2

from Fig. 1

Fig. 3

Antibody concentration(×10μg/ml)

Fig. 4

Peak 4

Peak 1

Peak 3

Peak 2

Retention Time (min.)

# Fig 5: A standard curve of competition ELISA

# Fig6: A result of Western blotting analysis

Lane  1: authentic IGF-II (20 ng)
         2: serum-derived IGF-II (normal adult)
         3: authentic IGF-I (1,000 ng)
         4: tumor tissue-derived IGF-II
         5: serum-derived IGF-II (gastric cancer and hypoglycemia)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 135 094 (AMGEN)<br><br>* page 27, line 11 - line 29; claims 3,36,42 *<br>--- | 1,4,5,<br>14,15 | C12P21/08<br>C12N15/06<br>C12N5/20<br>G01N33/577 |
| X | DOMESTIC ANIMAL ENDOCRINOLOGY<br>vol. 5, no. 4, October 1988, AUBURN, USA<br>pages 323 - 329;<br>F. BUONOMO ET AL.: 'Determination of circulating levels of insulin-like growth factor II (IGF-II) in swine.'<br>* abstract *<br>* page 325, line 3 - line 18 *<br>--- | 1,14,15 | G01N33/74 |
| A | EP-A-0 292 656 (MALLINCKBRODT, INC.)<br>* claims *<br><br>----- | 1-15 | |

| | | | TECHNICAL FIELDS SEARCHED (Int. Cl.5 ) |
|---|---|---|---|
| | | | C07K |

The present search report has been drawn up for all claims

| Place of search | Date of completion of the search | Examiner |
|---|---|---|
| THE HAGUE | 11 MARCH 1992 | NOOIJ F.J.M. |

EPO FORM 1503 03.82 (P0401)


SEQUENCE LISTING

(2) INFORMATION FOR SEQ ID NO:1:

    (i) SEQUENCE CHARACTERISTICS:
        (A) LENGTH: 14 amino acids
        (B) TYPE: amino acid
        (D) TOPOLOGY: linear

(ii) MOLECULE TYPE: peptide

(xi) SEQUENCE DESCRIPTION: SEQ ID NO:1:

```
Phe Ser Arg Pro Ala Ser Arg Val Ser Arg Arg Ser Arg Gly
1               5                   10
```

(2) INFORMATION FOR SEQ ID NO:2:

    (i) SEQUENCE CHARACTERISTICS:
        (A) LENGTH: 67 amino acids
        (B) TYPE: amino acid
        (D) TOPOLOGY: linear

(ii) MOLECULE TYPE: peptide

(xi) SEQUENCE DESCRIPTION: SEQ ID NO:2:

```
Ala Tyr Arg Pro Ser Glu Thr Léu Cys Gly Gly Glu Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Leu Glu Glu Cys Cys Phe
            35                  40                  45

Arg Ser Cys Asp Leu Ala Leu Leu Glu Thr Tyr Cys Ala Thr Pro Ala
    50                  55                  60

Lys Ser Glu
65
```

(2) INFORMATION FOR SEQ ID NO:3:

    (i) SEQUENCE CHARACTERISTICS:
        (A) LENGTH: 17 base pairs
        (B) TYPE: nucleic acid
        (C) STRANDEDNESS: single
        (D) TOPOLOGY: linear

(ii) MOLECULE TYPE: Other nucleotide (synthetic DNA)

(xi) SEQUENCE DESCRIPTION: SEQ ID NO:3:

TTCTTCGAGG ATACCTC                                                        17

(2) INFORMATION FOR SEQ ID NO:4:

    (i) SEQUENCE CHARACTERISTICS:
        (A) LENGTH: 8 amino acids
        (B) TYPE: amino acid
        (D) TOPOLOGY: linear

    (ii) MOLECULE TYPE: peptide


    (xi) SEQUENCE DESCRIPTION: SEQ ID NO:4:

    Ala Tyr Arg Pro Ser Glu Thr Leu
    1           5